# EUROPEAN PATENT SPECIFICATION

(11) **EP 2 583 979 B1**
(45) Date of publication and mention of the grant of the patent: **16.12.2015**
(21) Application number: 11306352.3
(22) Date of filing: 19.10.2011
(51) Int. Cl.: C07K 16/00, C07K 16/24

(54) **Methods to prepare antibodies directed against p19 subunit of human IL-23**
Verfahren zur Vorbereitung von menschlichem IL-23 gerichtete Antikörper, gegen die p19
Méthodes de preparation d'anticorps dirigés contre la sous-unité p19 de l'IL-23 humaine

(43) Date of publication of application: 24.04.2013
(73) Proprietor: Effimune, 44035 Nantes Cedex (FR); Institut National de la Santé et de la Recherche Médicale, 75654 Paris Cedex 13 (FR); Universite D'Angers, 49035 Angers Cedex (FR)
(72) Inventor: CHEVALIER, Sylvie, 49100 ANGERS (FR); DANGER, Yannick, 49440 LA CORNUAILLE (FR); FROGER, Josy-Anne, 49370 SAINT CLEMENT DE LA PLACE (FR); GASCAN, Hugues, 49100 ANGERS (FR); MARY, Caroline, 44680 SAINTE PAZANNE (FR); POIRIER, Nicolas, 44119 TREILLIERES (FR); VANHOVE, Bernard, 44400 REZE (FR)
(74) Representative: Desaix, Anne

(56) References cited:
- WO-A1-2008/103432
- WO-A1-2008/103473
- WO-A1-2010/115786
- WO-A1-2011/056600
- WO-A2-2007/005955
- WO-A2-2007/024846
- WO-A2-2007/027714
- WO-A2-2007/147019
- DATABASE UniParc [Online] 11 April 2011 (2011-04-11), "Sequence UPI0001EB060F", XP002671927, retrieved from UniProt Database accession no. UPI0001EB060F
- DATABASE UniParc [Online] 9 December 2009 (2009-12-09), "Sequence UPI000127C087", XP002671928, retrieved from UniProt Database accession no. UPI000127C087
- CHEN YI ET AL: "Anti-IL-23 therapy inhibits multiple inflammatory pathways and ameliorates autoimmune encephalomyelitis", JOURNAL OF CLINICAL INVESTIGATION,, vol. 116, no. 5, 1 May 2006 (2006-05-01), pages 1317-1326, XP002630082, DOI: 10.1172/JCI25308

## Description

The invention relates to methods for the preparation of antibodies directed against p19 subunit of human interleukin 23 (human IL-23) in its native form and have neutralizing capacity for the native human IL-23 protein. Also disclosed is the use of said antibodies in the preparation of therapeutic molecules.

The invention concerns in particular methods for the preparation antibodies, including their functional fragments or molecules including such antibodies or fragments which are functionally active, which exhibit neutralising properties with respect to native form of human IL-23 protein, sufficient to consider using these antibodies as drug candidates or active ingredients in therapeutic treatment of disorders involving pro-inflammatory cytokines pathways, especially involving interleukin IL-23 pathway, in Human. IL-23 is a member of the IL-12 cytokine family and is one of the regulators of T helper lymphocytes influencing immune response in disorders related to said immune response, such as autoimmune disorders, inflammatory disorders or in some cases pathogen infections.

Two forms of CD4+ helper T cells have been described for their pivotal role in orchestrating immune responses through their ability to assist other leukocytes involved in said responses. Th1 cells produce interferon gamma and interleukin 12 (IL-12) and are involved in immune cellular response, including responses to intracellular pathogens, and Th2 cells, predominantly secreting interleukin-4 (IL-4) contribute to humoral responses, allergic disorders and protection against gastrointestinal nematodes.

More recently a new subset of helper T lymphocytes, Th17 cells, was identified, in murine models and then in human. Th17 cells produce high levels of IL-17 and have shown to be able to keep immune hyperactivity (Cua DJ. Et al Annu Rev Immunol. 2007;25:221-42 Review).

Recent publications have evidenced the major involvement of the Th17 pathway in the development of experimental autoimmune disease models, i.e., experimental autoimmune encephalomyelitis, collagen-induced arthritis, psoriasis and inflammatory bowel disease (Cua DJ. et al Nature. 2003 Feb 13; 421 (6924): 744-8; Sato K. et al J Exp. Med. 2006 Nov 27; 203 (12): 2673-82; Izcue A. et al Immunity. 2008 Apr; 28(4):559-70; Uhlig HH. Et al Immunity 2006 Aug;25(2): 309-18; Chen Y. et al J. Clin. Invest. 2006 May; 116(5): 1317-26; Langrish CL. et al Immunol. Rev. 2004 Dec; 202:96-105). Results obtained using either p19/IL-23 (p19 is a protein characterised as one of the subunits of IL-23) deficient mouse strains or neutralizing antibodies directed against the mouse cytokine led to impressive results with complete disease remission, establishing IL-23 as an important mediator in chronic tissue inflammation.

During the last four years, several studies including in inventors' ones, underlined the importance of Th17 cells and IL-23 in chronic inflammation in patients (Pène J. et al J Immunol. 2008 Jun 1;180(11):7423-30; Annunziato F. et al J Exp Med. 2007 Aug 6;204(8):1849-61; Krueger GG et al N Engl J Med. 2007 Feb 8;356(6):580-92; Kauffman CL et al J Invest Dermatol. 2004 Dec;123(6):1037-44). This was particularly evidenced in psoriasis, Crohn's disease and rheumatoid arthritis.

IL-23 and IL-12 are produced in response to infections or inflammatory stimuli (Lyakh L.et al Immunol Rev. 2008 Dec; 226:112-31. Review). Products of microorganisms including bacteria, virus and fungi are also strong inducers of IL-23 secretion in macrophages, neutrophils and dendritic cells.

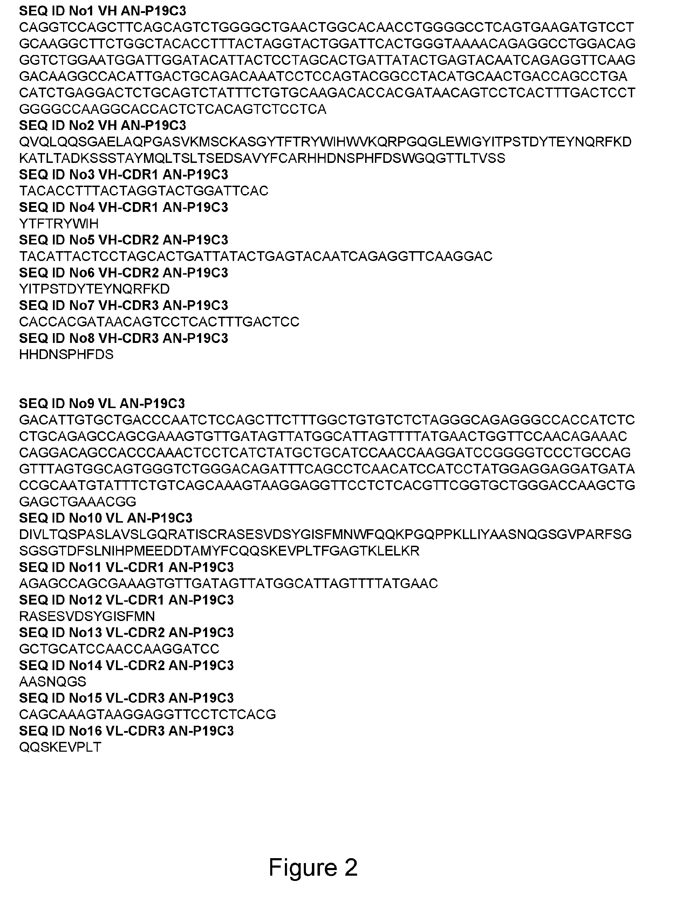

IL-23 is a heterodimer composed of a cytokine subunit, p19, associated to p40, a soluble cytokine receptor type protein. P40 also contributes to the formation of IL-12 when combined to p35 cytokine moiety. IL-12 and IL-23 are important mediators of Th1 and Th17 responses, respectively. Differences in the biological responses to IL-12 and IL-23 are in part explained by the recruitment of different membrane receptor complexes (figure 2).

The IL-12 receptor is made of two sub-units, IL-12Rβ1 and IL-12Rβ2, which display some homology with the IL-6 family signalling receptor, gp130 (Lupardus PJ. Et al J Mol Biol. 2008 Oct17;382(4):931-41; Wang X.et al Annu Rev Immunol. 2009;27:29-60. Review). IL-12Rβ1 binds the p40 subunit, while IL-12Rβ2 chain binds to p35. IL-12Rβ1-IL-12Rβ2 receptor complex signals through the recruitment of the Janus kinase 1 and Tyk 2 leading to phosphorylation of STAT1,-3,-4,-5, with a main role attributed to STAT4 activation.

IL-23 recruits the IL-12Rβ1 shared receptor component paired to a unique receptor subunit, the IL-23R. IL-23 signalling is related to the IL-12 one; but STAT3 is central in IL-23 signal transduction (Parham C. et al J Immunol. 2002 Jun 1;168(11):5699-708).

IL-12 is a more potent inducer of IFNγ-producing cells compared to IL-23. However, the most important activity of IL-23, that distinguishes it from IL-12, is its ability to induce the secretion of IL-17, and the expansion of Th17 cells (Boniface K et al. Immunol Rev. 2008 Dec;226:132-46 Review). Th17 cells were shown to express a pro-inflammatory cytokine signature profile consisting, besides IL-17, of IL-17F, IL-22, IL-26, CCL20/MIP3α. This human T cell subset also secretes high amount of IL-6 and TNFα (Pène J. et al 2008; Wilson NJ. et al Nat Immunol. 2007 Sep;8(9):950-7).

This subgroup of cytokines has major pro-inflammatory effects and contributes to tissue damage in autoimmune and inflammatory diseases.

IL-12 pathway plays a major role in the protection against intracellular pathogens. An increased susceptibility to mycobacteria, but also to salmonella infections was observed in patients displaying genetic defects in p40 or IL-12Rβ1 genes, reinforcing the idea that besides chronic inflammation the p40/p35 circuit is essential for immune protection against pathogens (Altare F. et al J Clin Invest. 1998 Dec 15;102(12):2035-40; Altare F. et al Science 1998 May 29;280(5368):1432-5; Filipe-Santos O et al. Semin Immunol. 2006 Dec;18(6):347-61).

Taking into account the importance of Th17 cells in the development and maintenance of chronic autoimmune diseases, it became an important challenge to develop antibodies that could specifically neutralize IL-23, without affecting the IL-12 pathway, especially to preserve its efficacy against pathogens.

Anti-IL-12 and anti-IL-23 would offer a welcomed and much needed therapeutic option for both anti-TNF primary and secondary nonresponders. Ustekinumab (CNTO-1275) and Brakinumab (ABT-874) are fully human monoclonal immunoglobulins targeting the interleukin IL-12/23 shared p40 subunit (Gandhi M. et al Semin Cutan ed Surg.2010 Mar;29(1):48-52 Review) In 2009, Ustekininumab has been approved by the US FDA and the European Medecines Agency for the treatment of moderate *to severe psoriasis.* Clinical trials also indicate some possible efficacy in psoriasis arthritis and in Crohn's disease. Nevertheless, some adverse effects were observed by targeting the p40 subunit. The most common adverse events were reported as upper respiratory tract infections, nasopharyngitis, headache and arthralgia (Marodi L. et al Nat Rev Immunol. 2010 May; 10(5):299-300).

Accordingly despite advances provided by these therapeutic antibodies in the treatment of specific diseases a major need still exists for the development of new drugs which would be capable of targeting IL-23 without affecting the IL-12 immune response. Such drugs should increase safety level of products, and increase the specificity of the neutralization towards chronic inflammation.

The present application discloses products that fulfil these needs at least in part. The inventors have indeed obtained antibodies, especially monoclonal antibodies targeting p19 subunit of IL-23 cytokine which exhibit neutralising capacity of native form of human IL-23, the structure of which enabled the design of derived polypeptides or complex molecules having the desired properties.

The antibodies of the invention were prepared using a different approach compared to the approach exemplified for preparation routes in the prior art reflected in the following cited patent applications. One difference in the approach according to the invention originates from the structure of the II-23 cytokine used as immunogen to obtain antibodies binding the p19 subunit of human II-23. Having indeed observed results disclosed in relation to neutralizing properties of prior prepared antibodies, the present inventors found that these prior antibodies would not be suitable with a view to design an active ingredient for a drug intended for administration effective and safe in human.

Hence, the inventors decided to select novel antibodies based on high through-put screening of generated hybridomas following immunisation of animals with native form of IL-23, especially human IL-23.

Native IL-23 displays a specific structural feature since its constitutive subunits, p19 and p40, are covalently linked through disulfide bridges. The impact of this feature was not considered by several groups attempting to prepare anti II-23 antibodies, which equally proposed to use different modified forms of IL-23 as an immunogen and in particular used recombinant fusion version of IL-23, adding a peptide linker between p19 and p40 subunits to generate a fused IL-23 used as immunogen. This led to a fused IL-23 easy to use and with a good bioactivity however generating, after immunization, antibodies having properties such that their effectiveness would require the administration of doses that would preclude their use as therapeutic candidates.

The use of such a fusion II-23 protein resulting from fusion of subunits p19 and p40 was illustrated especially in WO 2007/027714 T describing mAbs directed against p19.Table 6 of this patent application (p.55, p.56) illustrates the use of antibodies against fusion II-23 protein (elastikin), with a 450-fold increase of I.C.₅₀ required to neutralize the native form of IL-23 as compared to the fused cytokine. Similarly, a recent publication (Tonel G. et al J Immunol. 2010 Nov 15;185(10):5688-91) reported the capacity for humanized antibody 7G10 to neutralize psoriasis in a mouse xeno-transplantation model when injected at the high concentration of 50 mg/kg.

Likewise, in WO 2007/024846 anti-p19/IL-23 antibodies are disclosed that were also generated using the IL-23 fusion protein from R&D Systems (see examples 1 and 2). WO2011/056600 also discloses antibodies raised against II-23, but does not discuss the use of the native form IL-23.

Having identified the failure of the thus disclosed antibodies to provide suitable functional properties in terms of neutralisation capacity when administered in doses compatible with therapeutic regimen in an animal and accordingly in a human patient, the inventors have raised the hypothesis that the poor efficacy of said prior antibodies might be related to the structure of the IL-23 used for immunisation.

Having generated a number of mAbs recognizing p19 in the context of a fused single chain protein IL23 protein (fusion protein), the inventors concluded mAbs were unable, or only weakly able, to recognize the IL-23 native form. Among parameters influencing the properties of monoclonal antibodies they considered that the substantial differences in the structure of fused II-23 protein compared to the native structure of the cytokine might influence significantly the functionality of the antibodies produced as far as their neutralisation efficacy of native IL-23 was concerned. They then decided to immunize mice with the unmodified form (i.e. native form) of IL-23 to generate antibodies capable of efficiently interacting with the native form of the molecule and neutralise the same at doses that would allow contemplating their use as drug candidates.

The application accordingly discloses an antibody or a functional fragment thereof, which:
a) is obtainable from hybridoma cells prepared using spleen cells from an animal previously immunized with native human IL-23 protein as an immunogen , said spleen cells being fused with myeloma cells or,
b) is obtainable or is obtained from hybridoma AN-P19C3 deposited at the CNCM under No I-4539 on October 11, 2011.,
c) is expressed by recombinant eukaryotic cells which are recombined with nucleic acid molecule(s) identical to cDNA corresponding to RNA expressed in hybridoma AN-P19C3 deposited at the CNCM under No I-4539 that encodes an antibody of a) or b) or,
d) is a modified antibody with respect to a) or b) or c), having modified CDR regions in its Variable Heavy chain (VH) and/or in its Variable Light chain (VL), in particular keeping optionally at least one identical CDR3, CDR2 and/or CDR1 region in either of VH or VL or both, and/or having modified Framework (FR) and/or constant (CH) regions, said modified antibody having more than 70% identity, especially more than 75%, more than 80%, more then 85%, more than 90%, more than 95% or up to 99% identity over the whole length of its amino acid sequence, with the antibody of a) or b) or c), or,
e) is a functional fragment of a) or b) or c) or d); and
wherein said antibody or functional fragment thereof binds, in particular specifically binds, a protein which is the p19 subunit of native human IL-23 protein and said antibody or functional fragment thereof has neutralizing capacity for said native human IL-23 protein.

Native form of IL-23 cytokine has been described (Oppmann et al, Immunity. 2000 Nov;13(5):715-25) and made available in the prior art and can especially be obtained from commercial sources such as eBioscience (San Diego, CA, USA). Except otherwise stated when reference is made to IL-23 in the present application, it applies to human IL-23, in particular to native human IL-23. A recombinant form of IL-23, in its single-chain format, is also commercially available (Enzo Life Science, Villeurbanne, France or R&D Systems, Minneapolis, MN, USA) and although presenting the biological activity of native IL-23, it is structurally different. Unless otherwise specified, the term "p19 "herein refers to the human polypeptide having the sequence disclosed as NCBI, locus AAQ89442.

A *"functional fragment*" of an antibody as disclosed herein is a part of the antibody, i.e. a molecule corresponding to a portion of the structure of the antibody of the invention that exhibits antigen-binding capacity for the p19 subunit in native human IL-23; such fragment especially exhibits substantially the same antigen-binding capacity for said subunit when present in the native form of IL-23 as the antigen-binding capacity of the antibody. The antigen-binding capacity can be determined by measuring the affinity of the antibody and of the considered fragment.

Functional fragments of antibodies are fragments which comprise their hypervariable domains designated CDRs (Complementary Determining Regions) or part(s) thereof encompassing the recognition site for the antigen, i.e., p19 of native human IL-23, thereby defining antigen recognition specificity. Each Light and Heavy chain (respectively VL and VH) of a four-chain immunoglobulin has three CDRs, designated VL-CDR1, VL-CDR2, VL-CDR3 and VH-CDR1, VH-CDR2, VH-CDR3, respectively. Thus the application discloses fragments of antibodies of the invention, which comprise or consist in all or a selection of CDRs among VL-CDR1, VL-CDR2, VL-CDR3 and VH-CDR1, VH-CDR2 and VH-CDR3 or functional portions thereof, i.e. portions that exhibit the desired binding capacity preferably with a high affinity for p19 of native human IL-23.

Fragments that comprise or consist in VH-CDR3 and/or VL-CDR3 or functional portions thereof are especially preferred when CDR3 regions appear to be determinant in antigen recognition specificity.

The skilled person will be able to determine the location of the various regions/domains of antibodies by reference to the standard definitions in this respect, including a reference numbering system [Martin, A.C.R. (2001) Protein Sequence and Structure Analysis of Antibody Variable Domains. In: Antibody Engineering Lab Manual, ed.: Duebel, S. and Kontermann, R., Springer-Verlag, Heidelberg], or by reference to the numbering system of Kabat (Sequences of Proteins of Immunological Interest, 4th Ed., U.S. Department of Health and Human Services, NIH, 1987) or by application of the IMGT "collier de perle" algorithm (http://www.imgt.org/IMGTindex/Colliers.html). In this respect, for the definition of the sequences of the invention, it is noted that the delimitation of the regions/domains may vary from one reference system to another. Accordingly, the regions/domains as defined in the present invention encompass sequences showing variations in length or localization of the concerned sequences within the full-length sequence of the variable domains of the antibodies, of approximately +/- 10%.

Based on the structure of four-chain immunoglobulins, functional fragments can thus be defined by comparison with sequences of antibodies in accordance with the available data, especially with respect to comparison means and conventions cited above, and especially by comparison of the location of the functional domains in these sequences, noting that the positions of the framework and constant domains are well defined for various classes of antibodies, especially for IgGs, in particular for mammalian IgGs.

For illustration purpose of specific embodiments of the invention, antigen-binding fragments of an antibody that contain the variable domains comprising the CDRs of said antibody encompass Fv, dsFv, scFv, Fab, Fab', F(ab')2 which are well defined with reference to Kabat and also Roitt I. et al (Fundamental and Applied Immunology MEDSI/McGraw-Hill). Fv fragments consist of the VL and VH domains of an antibody associated together by hydrophobic interactions; in dsFv fragments, the VH:VL heterodimer is stabilised by a disulphide bond; in scFv fragments, the VL and VH domains are connected to one another via a flexible peptide linker thus forming a single-chain protein. Fab fragments are monomeric fragments obtainable by papain digestion of an antibody; they comprise the entire L chain, and a VH-CH1 fragment of the H chain, bound together through a disulfide bond. The F(ab')2 fragment can be produced by pepsin digestion of an antibody below the hinge disulfide; it comprises two Fab' fragments, and additionally a portion of the hinge region of the immunoglobulin molecule. The Fab' fragments are obtainable from F(ab')2 fragments by cutting a disulfide bond in the hinge region. F(ab')2 fragments are divalent, i.e. they comprise two antigen-binding sites, like the native immunoglobulin molecule; on the other hand, Fv (a VH-VL dimmer constituting the variable part of Fab), dsFv, scFv, Fab, and Fab' fragments are monovalent, i.e. they comprise a single antigen-binding site.

These basic antigen-binding fragments of the invention can be combined together to obtain multivalent antigen-binding fragments, such as diabodies, tribodies or tetrabodies. These multivalent antigen-binding fragments are also part of the present invention."Hybridoma cells" according to the invention are cells generated from fusion of antibody producing cells (B Lymphocytes) from an animal previously immunized with a selected immunogen and fusion partner which are myeloma cells enabling to provide immortality to the resulting fusion cell. Myeloma cells and antibody producing cells (B cells such as splenocytes) can be of the same origin, and are eukaryotic cells in particular mammalian cells of the same animal. They can be alternatively of different origin, thus giving rise to an heterohybridoma. Myeloma cells such as SP2/O mouse cells (Hurwitz et al, Cell. 1980 Nov;22(2 Pt 2):349-59) or rat IR983F cells (Lebacq-Verheyden et al, Hybridoma. 1983;2(3):355-8) are chosen among cells that fail to produce immunoglobulins in order to enable the prepared hybridoma to secrete only monoclonal antibodies of the desired specificity.

Preparation of hybridoma suitable for carrying out the invention is performed according to conventional techniques. Embodiments are described in detail in the examples of the present application of which the particular disclosed features can be adapted to other cells used as fusion partners.

A particular hybridoma suitable for carrying out the invention, useful for the preparation of the antibodies or functional fragments thereof is one where the myeloma cell used as fusion partner is the SP2/O cell line and a particular hybridoma in this embodiment is ANp19C3 deposited according to the provisions of the Budapest Treaty, at the CNCM (Collection Nationale de Cultures de Microorganismes, Paris , France) on October 11, 2011 under No I-4539.

In view of the teaching provided by the present invention in relation to the properties of the antibodies obtained from hybridoma AN-P19C3, in order to express antibodies of the invention, the skilled person will be able to use alternative technologies such as expression libraries and expression systems, followed by selection of antibodies having the structure of those secreted by hybridoma AN-P19C3 and having its binding and neutralisation properties. cDNA libraries can adequately be prepared from the RNA expressed in hybridoma of the invention and the appropriate sequences selected and expressed.

The application thus discloses antibodies or functional fragments thereof that have been obtained using cDNA prepared from RNA obtained from hybridoma producing antibodies of the invention, such as AN-p19C3 and performing an amplification reaction, such as a PCR with selected primers or pool of primers specific for antibodies coding nucleic acids, which primers are available in the art in order to amplify the cDNA sequences specifically coding for the antibodies disclosed herein. Following specific amplification, the cDNA is inserted in producing cells, especially in eukaryotic cells, under the control of regulatory sequences (including promoter elements) for expression and it is expressed.

Accordingly, the application thus discloses antibodies which have the same amino acid variable sequences as the monoclonal antibody produced by hybridoma AN-P19C3.

The functional fragments of the antibody may be obtained starting from the antibody, especially by using enzymatic digestion according to well known techniques including papain or pepsin digestion, or using any appropriate cleavage technique. They may be alternatively expressed in host cells modified by recombination with nucleic acid sequences encoding the amino acid sequence of said fragments, or may be synthesized, especially chemically synthesized.

Accordingly, the antibodies as disclosed herein, including the modified antibodies, and the functional fragments of the antibodies can also be prepared by classical genetic engineering techniques, such as those described by Sambrook et al. [Molecular Cloning, A Laboratory Manual, 2nd Ed., Cold Spring Harbor Laboratory Press, Cold Spring Harbor, N. Y., (1989), and updated versions].

In accordance to the invention, "*binding*" to the p19 protein refers to an antigen-antibody type interaction and reference to "*specific binding*" properties of the antibodies or functional fragments thereof means that the antibodies or functional fragments thereof bind to the p19 subunit of human IL-23 and bind to the human IL-23, especially native form of human IL-23 and furthermore do not bind or bind with a significant weaker affinity to p40 subunit of human IL-23 and do not bind to human IL-12. Binding capacity can be assayed in accordance with the tests disclosed in the Examples and in particular can be assayed by ELISA, or Western Blot analysis. The binding capacity may be measured by determination of the binding affinity for native human IL-23 or p19.

Particular antibodies disclosed hereinare monoclonal antibodies that have an affinity in the range of 10⁻⁸ KD (M) to 10⁻¹⁰ KD (M) with reference to dissociation constant, especially has an affinity corresponding to a Kd (dissociation constant) of 1.4nM, when determined by Surface Plasmon Resonance for example with BIAcore Instrument. The assay is carried out on captured antibody or fragment thereof (by protein A or by anti-Fc antibody on a chip, and human IL-23 is flowed past. The mass formation at the surface of the chip is measured.

"*Neutralising properties*" or neutralising capacity of the antibodies or functional fragments thereof means that the antibodies or functional fragments thereof interfere or inhibit the biological effect of IL-23 on cells expressing IL-23 receptor. The neutralisation capacity of the antibodies disclosed herein or functional fragments thereof can de determined by the IC₅₀ value (i.e, the concentration of the tested antibody or fragment that induces half-maximal inhibition of IL-23 mediated cell proliferation).

In a particular embodiment neutralising capacity corresponds to the capacity of the antibodies or functional fragments thereof to inhibit the binding of human IL-23, especially native form of human IL-23, to its cellular receptor, IL-23R, thereby inhibiting STAT-3 phosphorylation. The ability to inhibit the binding of IL-23 to IL-23R can easily be tested using for instance, an *in vitro* assay using cells expressing IL-23R at their surface and growing in the presence of IL-23 only. Appropriate cells having these properties and suitable to perform the inhibition test are TF23 cells. Such cells were obtained easily by growing the TF1 cell line (a Human erythroleukemia cell line expressing the IL-12Rβ1 receptor and which proliferation is driven by GM-CSF or erythropoietin; available in the Collection ECACC under No 93022307 - Kitamura T, et al Cell Physiol. 1989 Aug;140(2):323-34.) in the presence of IL-23. The resulting cells then acquired the capacity to grow in the presence of IL-23, and the cell line was termed TF23. For proliferation assays, TF23 could then be seeded in 96-well plates at a concentration of 5-10x10³ cells/well in RPMI 1640 medium containing 5% FCS, in the presence of IL-23. An inhibitory antibody added to the reaction then broke proliferation, which was measured after a 5-day incubation period by addition of 0,5 µCi of ³HTdr to each well for the last 4 hours of the culture. The incorporated radioactivity was then determined by scintillation counting.

Also, in presence of an inhibitor of the binding of IL-23 to IL-23R, signal transduction and STAT-3 phosphorylation are suppressed in TF23 cells. The evaluation of the phosphorylation level of STAT-3 allows determination of the inhibitory properties. Examples of assays enabling detection of neutralisation capacity of the antibodies as disclosed herein or their functional fragments are disclosed in the Examples, especially using TF23 cells.

This inhibition capacity can also be detected as an inhibition of global cells proliferation of cells expressing the IL-23 receptor in proliferation bioassay (illustrated in the Examples with TF23 cells), using a dose/response curve with various concentrations of IL-23.

Particular antibodies disclosed hereinare antibodies having neutralising capacity in a range of IC₅₀ 10⁻⁹M when measured on native human IL-23. In a particular embodiment, such antibodies may further advantageously combine with this property, a binding affinity in the range disclosed above.

In the frame of the definition of the invention a modified antibody is an antibody having modified CDR region(s) in its Variable Heavy chain (VH) and/or in its Variable Light chain (VL), optionally keeping one non-modified CDR among CDR3, CDR2 and CDR1 regions in either of VH or VL or both, and/or having modified framework (FR) and/or constant (CH) regions, said modified antibody having more than 70% identity, especially more than 75%, more than 80%, more than 85%, more than 90%, more than 95% or up to 99% identity over the whole length of its amino acid sequence, with the antibody obtainable from or obtained from hybridoma AN-P19C3 deposited at the CNCM.

Particular examples of modified antibodies obtained according to the invention encompass chimeric antibodies, humanized antibodies and/or a de-immunized antibodies.

A particular modified antibody has modified amino acid residues in the CDRs regions, said modification resulting in a de-immunisation by loss of the T cell epitopes in the variable domain of the non-human antibody. De-immunisation can be performed after determination of the T cell epitopes in the antibody variable domain, especially by in silico prediction, followed by point mutation in the sequence of the variable chains of the antibody that eliminates the functional T cell epitopes. De-immunisation can especially concern amino acid residues in the framework region of the variable chains and be carried out according to Kim et al (Biochem Biophys Res Commun. 2010 May 28;396(2):231-7.

In a preferred embodiment of the invention, the modification of the CDR(s) regions, especially of the CDR3 regions are limited to the extent necessary to de-immunisation with a view to administration to the human body, e.g. to decrease binding affinity of derived peptides for HLA-class I or class II. T cells. In a particular embodiment, the CDR3 region(s) of the VH and/or of the VL is(are) not modified. In another embodiment the FR regions and/or the CH regions are also modified, especially humanized.

Antibodies obtained according to the invention encompass accordingly an antibody based on the hereabove defined features, which is a humanized antibody especially one obtained by substitution of amino acid residue(s) present in constant region(s) of an antibody disclosed herein, for human amino acid residue(s) having corresponding location in human antibodies according to standard definition and numbering, wherein the substitution level is from 1% to 18% of the residues in said constant regions CH and/or FR regions, and where appropriate for de-immunisation into some or all of the CDR(s) region(s).

As mentioned above, the humanization primarily targets the Framework regions of the original antibodies. In some cases, humanization may alternatively or also concern CDR region(s) especially CDR1 and/or CDR2 region(s).

Humanization can hence be achieved considering the human germline Light chain or Heavy chain frameworks that show the highest sequence identity with the sequence of the non-human antibody or fragment, and selecting the amino acid residues, especially residues exposed at the surface in the antibody, to be substituted in said non-human antibody or fragment thereof, in order to conform to the corresponding human residue(s). In a particular embodiment some of, or all, the FRL and/or some of, or all, the FRH regions are fully human, i.e., are characteristic of human framework sequences. In another embodiment selected residues in some of all the FR regions are substituted.

Methods for humanizing antibodies are also well known in the art and are described for instance by Routledege et al. ["Reshaping antibodies for therapy", in Protein Engineering of Antibody Molecules for Prophylactic and Therapeutic Applications in Man, 13-44, Academic Titles, Nottingham, England (1993)] or by Roguska et al. Protein Engineering, 9(10), 895-904, (1996)]. These methods can also apply to antigen-binding fragments, such as scFvs.

By way of example, the method known as "resurfacing" consists in replacing the set of surface residues in the frameworks of the variable region of a nonhuman antibody with a human set of surface residues, while the method known as CDR grafting consists of transferring the CDRs from a non-human antibody into the framework regions of a human antibody. CDR grafting is generally completed by framework optimization, consisting in the replacement of some residues of the human framework, in order to optimize the binding affinity.

The step of framework optimization has been recently simplified by the use of combinatorial libraries (Rosok. et al. J. Biol. Chem. 271, 22611-22618, 1996; Baca et al. J. Biol. Chem. 272, 10678-10684, 1997).
Another recent strategy available for antibody humanization preserves only the original nonhuman CDR3 sequences of light and heavy chain while the remaining sequence is selected from naïve human V gene libraries (Rader et al, Proc. Natl. Acad. ScL U.S.A. 95, 8910-8915, 1998).

According to another embodiment of the invention, the antibodies are modified and are, as a result, chimeric antibodies, comprising domains or strand(s) of amino acid residues of different antibodies, in particular antibodies obtained from different animal species, combined together in a functional antibody.

The chimeric, humanized or de-immunized antibodies disclosed herein can belong to any class of immunoglobulins, like the non-modified one. Preferably, they belong to a subclass of the IgG class such as IgG1, IgG2, IgG3 or IgG4.

Methods for preparing recombinant antigen-binding fragments, or chimeric antibodies by combining the variable regions of an antibody with appropriate linkers, or with the constant regions of another antibody, are well known in the art.

The antibodies disclosed herein are said to be monoclonal antibodies, meaning that a composition of these antibodies is homogeneous, especially identical, in terms of antigen-binding specificity and accordingly in terms of variable region composition. Hence the antibodies may qualify as monoclonal even if they are obtained by techniques alternative to the technique of hybridoma.

In a particular embodiment of the invention, an antibody or a functional fragment thereof, comprises an amino acid sequence having SEQ ID No 8 (VHCDR3) and which optionally further comprises an amino acid sequence having SEQ ID No 16 (VLCDR3). These fragments may be modified, especially de-immunised, including possibly by modifying amino acid residues in the CDR3 region(s), to the extent that the resulting fragments keeps sufficient binding capacity and neutralising capacity for the p19 subunit in native human IL-23.

In a more specific embodiment of the invention, the antibody or a functional fragment thereof comprises the following CDRs regions involved in the antigen-binding site:
a. for the Variable Heavy chain:
   i. CDR 1 having the amino acid sequence SEQ ID No 4
   ii. CDR 2 having the amino acid sequence SEQ ID No 6
   iii. CDR 3 having the amino acid sequence SEQ ID No 8
b. for the Variable Light chain:
   i. CDR 1 having the amino acid sequence SEQ ID No 12
   ii. CDR 2 having the amino acid sequence SEQ ID No 14
   iii. CDR 3 having the amino acid sequence SEQ ID No 16

In particular an antibody or a functional fragment thereof obtained according to the invention is one, whose variable domain comprises Variable Heavy chain polypeptides having amino acid sequence SEQ ID No 2 and/or Variable Light chain polypeptides having amino acid sequence SEQ ID No 10.

The one-letter symbols are used to disclose the amino acid residues of the amino acid sequences.

The thus defined fragments may be modified, especially humanized, de-immunised,or rendered chimeric according to the definitions provided herein, including possibly by modifying amino acid residues in the CDR(s) region(s), to the extent that the resulting fragments keeps sufficient binding capacity and neutralising capacity for the p19 subunit in native human IL-23.

A preferred embodiment disclosed herein relates to an antibody, which is obtained from hybridoma AN-P19C3 deposited at the CNCM under No I-4539 said antibody is designated AN-P19C3 in the following Examples.

A preferred modified antibody is one derived from this preferred antibody obtained from hybridoma AN-P19C3.

Preferred functional fragments of the antibody as disclosed herein and preferred modified functional fragments are those obtainable from antibody obtained from hybridoma AN-P19C3 or corresponding modified antibody.

The application also discloses a polypeptide which comprises an amino acid sequence having SEQ ID No 8 (VHCDR3).

Another polypeptide disclosed hereinis a polypeptide comprising an amino acid sequence having SEQ ID No 8, which further comprises an amino acid sequence having SEQ ID No 16 (VLCDR3).

In a particular embodiment such a polypeptide comprising an amino acid sequence having SEQ ID No 8 and further comprising an amino acid sequence having SEQ ID No 16, is a fragment of an antibody that binds a protein which is the p19 subunit of native human IL-23 protein and has neutralizing capacity for said native human IL-23 protein, wherein said fragment retains the binding capacity, especially the specific binding capacity, of said monoclonal antibody, toward the p19 subunit of native human IL-23 protein.

A preferred polypeptide of this group is a polypeptide that retains neutralizing capacity of the monoclonal antibody for said native human IL-23 protein.

According to another embodiment, the application also discloses a chimeric molecule which comprises an antibody according to any of the definition provided herein or a functional fragment thereof, wherein said monoclonal antibody or functional fragment thereof is associated with a functionally different molecule. A chimeric molecule as disclosed herein may be either a fusion chimeric protein or a conjugate resulting from any suitable form of attachment including covalent attachment, grafting, chemical bonding with a chemical or biological group or a molecule, such as a PEG polymer or another protective group or molecule suitable for protection against proteases cleavage *in vivo,* for improvement of stability and/or half-life of the antibody or functional fragment, with a biologically active molecule, especially a therapeutic active ingredient, a vector (including especially a protein vector) suitable for targeting the antibody or functional fragment to specific cells or tissues of the human body, or with a label or with a linker, especially when fragments of the antibody are used.

PEGylation of the antibody or functional fragments thereof is a particular interesting embodiment as it improves the delivery conditions of the active substance to the host, especially for a therapeutic application. PEGylation can be site specific to prevent interference with the recognition sites of the antibodies or functional fragments, and can be performed with high molecular weight PEG. PEGylation can be achieved through free Cysteine residues present in the sequence of the antibody or functional fragment or through added free Cysteine residues in the amino sequence of the antibody or functional fragment.

The application also discloses a composition comprising antibodies or functional fragments thereof as defined herein, wherein the antibodies or functional fragments thereof are a homogeneous population (recognizing the same p19 antigen subunit in the native human IL-23 protein) of antibodies or functional fragments thereof or are monoclonal antibodies or functional fragments thereof.

The definitions provided herein especially by reference to the antibodies disclosed herein, similarly apply to the functional fragments thereof except where it is technically obviously not relevant. These definitions also apply to molecules (in particular chimeric antibodies or chimeric molecules) or compositions comprising these antibodies or functional fragments thereof or derived from these antibodies, as disclosed in the present application. It is further specified that the functional fragments of the antibodies are derived from the antibodies from a conceptual point of view but may be prepared through various techniques, not necessarily having recourse to the antibodies as products.

The application also discloses a nucleic acid molecule encoding an antibody or a polypeptide as disclosed herein, especially a functional fragment of said antibody.

A particular nucleic acid disclosed herein is chosen in the group of:
a. A polynucleotide encoding the VH region and having the sequence of SEQ ID No 1,
b. A polynucleotide encoding the VL region and having the sequence of SEQ ID No 9,
c. A polynucleotide encoding the VHCDR1 region and having the sequence of SEQ ID No 3,
d. A polynucleotide encoding the VHCDR2 region and having the sequence of SEQ ID No 5,
e. A polynucleotide encoding the VHCDR3 region and having the sequence of SEQ ID No 7,
f. A polynucleotide encoding the VLCDR1 region and having the sequence of SEQ ID No 11,
g. A polynucleotide encoding the VLCDR2 region and having the sequence of SEQ ID No 13,
h. A polynucleotide encoding the VLCDR3 region and having the sequence of SEQ ID No 15,
i. A polynucleotide having modified nucleotides with respect to the sequence of SEQ ID No 1, or SEQ ID No 3, or SEQ ID No 5, or SEQ ID No 7, or SEQ ID No 9, or SEQ ID No 11, or SEQ ID No 13, or SEQ ID No 15, and encoding a polypeptide having amino acid sequence of respectively SEQ ID No 2, or SEQ ID No 4, or SEQ ID No 6, or SEQ ID No 8, or SEQ ID No 10, or SEQ ID No 12, or SEQ ID No 14, or SEQ ID No 16,
j. A polynucleotide having at least 85%, preferably at least 90%, more preferably at least 95%, and most preferably at least 98% or at least 99% identity over its whole length with one of the polynucleotides having sequence of SEQ ID No 1, or SEQ ID No 3, or SEQ ID No 5, or SEQ ID No 7, or SEQ ID No 9, or SEQ ID No 11, or SEQ ID No 13, or SEQ ID No 15,
k. A polynucleotide which is a fragment of the polynucleotide having sequence of SEQ ID No 1, or SEQ ID No 3, or SEQ ID No 5, or SEQ ID No 7, or SEQ ID No 9, or SEQ ID No 11, or SEQ ID No 13, or SEQ ID No 15 and which encodes a functional fragment of a polypeptide having amino acid sequence of respectively SEQ ID No 2, or SEQ ID No 4, or SEQ ID No 6, or SEQ ID No 8, or SEQ ID No 10, or SEQ ID No 12, or SEQ ID No 14, or SEQ ID No 16.

Polynucleotides disclosed herein , especially polynucleotides encoding the variable regions of the antibody ANp19C3 or the CDRs thereof can be, for example, obtained by cloning said regions from a cDNA library of the hybridoma deposited at the CNCM under No I-4539 They can also be prepared, completely or partially, by nucleic acid synthesis, based on the nucleotide sequences provided herein.

Polynucleotides disclosed herein can be optimized sequences, especially for the expression in host cells. Optimisation techniques in this field are conventional one.

Polynucleotide fragment of option k) above have advantageously a sequence of at least 9 nucleotides and are shorter than the original one.

According to a particular embodiment, polynucleotides disclosed herein may advantageously comprise, besides a sequence encoding an antibody or a functional fragment thereof, or a chimeric molecule including the same as disclosed herein, a sequence encoding a signal peptide allowing secretion of said protein. They may also comprise one or more sequence(s) encoding one or more marker peptide(s) for detecting, and/or facilitating the purification of, said protein.

The application also discloses a vector for the cloning and/or for the expression of a polynucleotide as defined herein, especially a plasmid suitable for cloning and/or expressing in cells, especially in mammalian cells, including in human cells. Expression vectors comprise as an insert, a polynucleotide of the invention, driven by transcription and translation control sequences selected for the host cell chosen for cloning and/or expression.

The application discloses a cell or a cell line recombined with a polynucleotide or a vector as defined herein, especially a prokaryotic or eukaryotic cell, in particular a mammalian cell or cell line.

Examples of prokaryotic host cells include bacteria such as *E.coli.* Among suitable eukaryotic cells, mention will in particular be made of plant cells (in the case of plantibodies), cells from yeast, such as Saccharomyces, Kluyveromyces, or Pichia pastoris, insect cells, such as Drosophila or Spodoptera cells, and mammalian cells such as HeLa, CHO, 3T3, C127, BHK5 Heck 293, COS, etc., cells.

The construction of expression vectors in accordance with the invention and the transformation of the host cells is carried out by the conventional techniques of molecular biology.

The application further discloses a spleen cell or a cell line obtained from a spleen cell, wherein said cell or cell line has been previously obtained from an animal immunized with human native IL-23.

A preferred cell line disclosed herein is a hybridoma, especially hybridoma AN-p19C3 deposited at the CNCM under No I-4539, or a heterohybridoma.

The invention also provides a method for the preparation of hybridoma expressing antibodies of the invention, which comprises the steps of immunizing an animal, especially a mammalian (mice, rat, rabbit...) with human IL-23 in its native form and, where necessary, boosting said animal with the same immunogen, recovering spleen cells from the animal responding to immunization and fusing said cells with myeloma cells in conditions enabling the recovery of hybridoma producing the antibodies having the desired binding affinity.

The invention thus further concerns a method for preparing antibodies according to the invention, comprising preparing hybridoma according to the above steps and recovering from the supernatant of the hybridoma the expressed monoclonal antibodies binding the p19 subunit in native human IL-23, and optionally purifying the same.

Another object disclosed herein is a library of cDNA obtained from the RNA of a hybridoma of the invention, especially of hybridoma ANp19C3.

The invention also concerns a method of expression of the antibody or functional fragment thereof according to the invention by selecting the cDNA from such a library and expressing this cDNA in a host cell, especially in a eukaryotic suitable for a modified glycosylation of the expressed polypeptides. Examples of eukaryotic cells suitable for a modified glycosylation are cells of avian origin, such as the EB66 cell line (Olivier et al, MAbs. 2010 Jul 16;2(4)), or CHO cells engineered for having a lower fucosyl transferase activity (von Horsten et al, Glycobiology. 2010 Dec;20(12):1607-18).

The invention also relates to a process for the preparation of antibodies according to the definitions defined herein, comprising the step of expressing polynucleotides of the invention in producing cells, especially in cells of the type mentioned above, in particular those permitting a modified glycosylation..

The application also discloses a pharmaceutical composition comprising an antibody or a functional fragment thereof or a chimeric molecule, as defined herein, associated with a pharmaceutical vehicle, wherein said pharmaceutical composition optionally further comprises a different therapeutically active ingredient.

The application discloses in particular an antibody or a functional fragment thereof or a molecule of the invention or a pharmaceutical composition, for the treatment of an inflammatory disease, especially chronic inflammatory diseases, such as rheumatoid arthritis, psoriasis, multiple sclerosis or Crohn's disease, another autoimmune disease or a situation such as cell or organ transplantation in a human patient in need thereof.

The treatment is carried out with effective doses of the antibodies of the invention or functional fragments thereof, and the regimen is adapted to the patient condition.

Agents that actively block TNFa such as Ethanercept (Enbrel, Amgen) downregulate pro-inflammatory pathways and are highly effective treatments in these indications. However, there remains a population of patients with reluctant diseases. For example, about a third of patients with moderate to severe psoriasis fail to respond to TNFa blockade. These patients diagnosed for reluctant disease might be advantageously treated with an antibody as disclosed here.

Instead of receiving an anti-TNFa treatment, it has been reported in the art that patients might also be treated with anti-IL-12/IL-23 p40 antibodies, such as Ustekinumab or Brakinumab. However, some adverse effects were observed by targeting p40 subunit, such as upper respiratory tract infections, nasopharyngitis, headhaches, arthralgia, that conducted investigators to withdraw the Brakinumab approval application (Gandhi et al, Semin Cutan Med Surg. (2010) 29(1): 48-52 and Marodi et al, Nat Rev Immunol (2010); 10(5):299-300. These adverse events are presumably caused by the unwanted neutralization of IL-12, in addition to the neutralization of IL-23, which prevents patient's immune system from fighting against pathogenic viruses. In a particular embodiment disclosed herein, antibodies as disclosed herein are administered to patients previously treated with anti-p40 antibodies who experienced adverse effects or are administered instead of anti-p40 antibodies to prevent occurancy of such adverse effects.

In a particular embodiment, the antibodies disclosed hereinor functional fragments thereof are administered as the sole component to the patient. Alternatively, the antibodies dislclosed herein or functional fragments thereof are included in a combination treatment with further therapeutically active ingredients. According to another embodiment, the antibodies disclosed herein or functional fragments thereof are administered as an add-on treatment, following the administration of a first therapeutically active ingredient whose administration is then discontinued or continued.

By "*treatment*" or "*therapeutic treatment*", it is meant that the performed steps of administration result in improving the clinical condition of an animal or a human patient in need thereof, who suffers from disorder(s) associated with the IL-23 pathway. Such treatment aims at improving the clinical status of the animal or human patient, by eliminating or lowering the symptoms associated with the disorder(s) related to the IL-23 pathway and/or in a preferred embodiment, restoring to health.

The efficiency of the disclosed products of the application, especially antibodies as disclosed herein or functional fragments thereof in therapeutic treatment can be assessed on animal models, especially murine models presenting pathogenic autoimmune and inflammatory conditions, such as experimental encephalomyelitis, collagen-induced arthritis, or intestinal inflammation.

In particular Dextran Sulfate Sodium (DSS)-induced acute colitis model of inflammation disease may be used as it has been shown that in this model IL-23 is able to cross-regulate IL-12 production. Accordingly, acute colitis is induced by feeding mice with 5% (wt/vol) DSS dissolved in drinking water, *ad libitum* for 7 days. The progression of the disease is monitored daily by measuring, weight loss, checking the significance appearance of diarrhea/loose faeces, and the presence of visible faecal blood. To assess the potential effects of candidate molecules as disclosed herein on the pathology, inflammatory gene expression levels in colon and cytokine protein levels in sera are quantified using real-time PCR and multiplex technology (Pène J. et al 2008).

A model of autoimmune disorder exists for Psoriasis which is a common T cell-mediated autoimmune disorder where primary onset of skin lesion is followed by chronic relapses. A mouse model is used in which skin lesions spontaneously develop when symptomless pre-psoriatic human skin was engrafted onto AGR129 mice, deficient in type I and II interferon receptors and for RAG2 (Tonel G. et al 2010) or in SCID/Nod mice. Upon engraftment, resident human T cells in pre-psoriatic skin undergo local proliferation. Th17 cell development is crucial for a development of a psoriatic phenotype in this model. Injections of the selected candidate molecule of the invention are performed in this model and the effects of the injections are monitored.

Further aspects and features of the invention will be apparent from the examples which follow and from the figures.
Figure 1 describes the antibodies prepared using various subunits of IL-23 antigen.
Figure 2 describes the compared structures of IL-12 and IL-23
Figure 3 describes binding of the antibody anti-p19/IL-23 (ANp19C3) revealed using ABTS [2,2'-azino-bis(3- ethylbenzthiazoline-6-sulfonic acid)] as a substrate, after reading carried out at 405 nm. The results show that AN-p19C3 specifically binds p19 and IL-23, and does not bind p40 or the control protein, soluble CNTFR.
Figure 4 describes Western Blot analysis performed with the antibody anti-p19/IL-23 (ANp19C3). The results show that the AN-p19C3 recognized p19 alone or in the IL-23 context. In contrast, no signal is observed for p40 or IL-12.
Figure 5 describes a dose/response curve of TF23 cells treated with IL-23 showing that cells proliferate in the presence of the cytokine
Figure 6 describes the inhibition by ANp19C3 antibody on the proliferation of TF23 cell line induced by IL-23. A typical neutralization effect of AN-p19C3 antibody towards IL-23 is shown and it is also shown that another antibody against p19 did not neutralize IL-23 to the same extent.
Figure 7 describes the results of tyrosine phosphorylation analysis. Figure 7 shows a typical neutralization effect of AN-p19C3 antibody towards STAT-3 phosphorylation in TF23 cells.
Figure 8 describes the affinity for IL-23 measured for ANp19C3 antibody. The concentrations of IL-23 assayed were 24, 74, 222, 667 and 2000 nM. No binding was observed with the IL-12 molecules (data not shown) whereas a 12 nM affinity was measured for IL-23.

### Experimental data

### PRODUCTION AND CHARACTERIZATION OF THE ANTI-P19/IL-23 ANTIBODY AN-P19C3

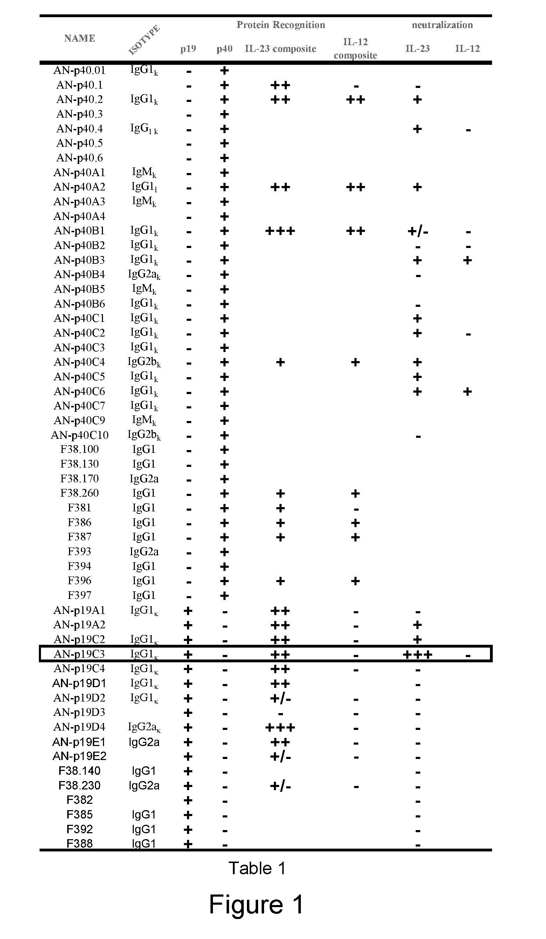

Using their high-troughput antibody platform, the inventors developed more than 55 different monoclonal antibodies raised against either p40 or p19 subunits of IL-23 (table 1 on Figure 1).

A number of generated antibodies recognized p40 and were good neutralizing tools for both IL-12 and IL-23. It was much more challenging to generate antibodies directed against p19. Indeed, p19 is clearly a weakly antigenic protein, rendering the immunization processes to generate mAbs a lot more complicated.

The use of a fused form of IL-23 (p19-p40 or p40-p19 expressed fused cytokines), as earlier performed by others, led the inventors to the generation of anti-p19 mAbs, that only or mainly recognized p19 subunit in the context of the fused protein p40-p19. But these same anti-p19 antibodies failed, or only very weakly recognized, the native form of heterodimer, rendering these antibodies non suitable for any further use.

However the inventors have succeeded in obtaining monoclonal antibodies able to specifically recognize p19 when naturally associated (i.e., through a disulfure bridge) to p40 and which antibodies showed neutralizing properties for IL-23 response without affecting the IL-12 response. IL-23 in its native form used for immunization was the recombinant protein in its native form from eBioscience (ref34-8239) and it was injected as 1 µg per injection. 4 intrasplenic injections with an interval of 15 days between each were performed.

Among the 17 anti-p19 mAbs obtained in mice, only three were considered neutralizing, with one showing particularly effective capacity in efficiently blocking IL-23 bioactivity (clone AN-P19C3).

This monoclonal antibody, hereinafter designated " ANp19C3" or also "AN-P19C3" was produced by the hybridoma deposited in accordance with the terms of Budapest Treaty, at the CNCM (Collection Nationale de Cultures de Microorganismes, Institut Pasteur, 25 rue du Docteur Roux, 75724 Paris Cedex 15, France), on October 11, 2011, under the deposit number I-4539.

The inventors have cloned and sequenced the variable domain (VL) of the light chain, and the variable domain (VH) of the heavy chain of monoclonal antibody ANp19C3. The limits of the sequences encoding the complementarity determining regions (CDRs) of said antibody have been obtained, classically, by aligning these VH and VL sequences against the IMGT reference database (Lefranc et al., Nucl. Acids Res., 33, Database issue D593-597, 2005), using the software program IMGT/V-QUEST (Giudicelli et al. Nucl. Acids Res., 32, Web Server issueW435-440, 2004). These sequences are described below in Table 2 for the heavy chain and for the light chain.

### Molecular cloning of AN-p19 C3 CDRs

### Preparation of RNA and cDNA synthesis

RNA was prepared from about 6 x 10⁶ hybridoma cells AN-P19C3. RNA was extracted with Rneasy mini kit (Qiagen).

Oligo dT primed cDNA was obtained by reverse transcription of 1µg of total RNA with Superscript II reverse transcriptase (Invitrogen) in 20 µL reaction.The resulting cDNA was purified with Qiaquick PCR purification kit (Qiagen) and directly used for amplification.

### PCR amplification of VH and VL

For amplification of VH and VL 2 µL cDNA was subjected to 35 cycles of PCR using Taq Triple Master high fidelity (Eppendorf) in two separate tubes.

The following components were added to the cDNA: 5 µL 10X buffer, 1 µL 10mM dNTPs, 0,5 µL Taq DNA polymerase, and 1 µL of this primers:
IgG1-REV: 5'-GGAAGGTGTGCACACTGCTGGAC-3'
MsVHE-FOR: 5'-GGGAATTCGAGGTGCAGCTGCAGGAGTCTGG -3' for VH and,
kappa-REV: 5'-GGATACAGTTGGTGCAGCATC -3'
kappa-FOR: 5'-GAYATTGTGMTSACMCARWCTMCA-3' (in which; M = A or C; R = A or G; S = C or G W = A or T) for VL.
PCR was performed using the following amplification protocol: 94°C for 2 min followed by 35 cycles of 94°C for 20 sec, 60°C for 30 sec and 72°C for 1min then 8 µL were taken and analyzed on 1,5% agarose gel.

### Sequencing of VH and VL

For sequencing, each PCR product was cloned into pGEMT-easy (Promega) according to the manufacturer's instructions. Individual clones were sequenced using the CEQ^{™}8000 (Beckman Coulter) with forward and reverse sequencing primers of the vector and the cloning primers.
Sequences were analyzed on IMGT web site to determinate VH, VL and their CDR regions. They are disclosed in Table 2 which follows.

| Sequence ID | Domain | Sequence |
|---|---|---|
| 1 | VH | |
| | | |
| 2 | VH | |
| 3 | VH-CDR1 | TACACCTTTACTAGGTACTGGATTCAC |
| 4 | VH-CDR1 | YTFTRYWIH |
| 5 | VH-CDR2 | |
| 6 | VH-CDR2 | YITPSTDYTEYNQRFKD |
| 7 | VH-CDR3 | CACCACGATAACAGTCCTCACTTTGACTCC |
| 8 | VH-CDR3 | HHDNSPHFDS |
| 9 | VL | |
| 10 | VL | |
| 11 | VL-CDR1 | |
| 12 | VL-CDR1 | RASESVDSYGISFMN |
| 13 | VL-CDR2 | GCTGCATCCAACCAAGGATCC |
| 14 | VL-CDR2 | AASNQGS |
| 15 | VL-CDR3 | CAGCAAAGTAAGGAGGTTCCTCTCACG |
| 16 | VL-CDR3 | QQSKEVPLT |

In the above table, the location (position) of the CDRs regions has been disclosed with respect to the Kabat convention system.

### ELISA detection:

The AN-p19C3 binding properties produced were tested by ELISA. Antihuman IgG (Fc specific- Sigma-Aldrich : 12136) was coated at a concentration of 5 µg/ml in 50 mM carbonate buffer, pH 9.6 (5h, 37°C), in 96-well boxes. After washes and a Tris 0.1 M sucrose 20% saturation step (1h30, room temperature), p19Fc or p40Fc or p19Fc-p40flag cytokines were added to the wells at a final concentration of 100 ng/ml. In part of the wells a same amount of CNTFR-Fc protein was added as an irrelevant protein to be able to reinforce the specific component of measured signals. After one night of contact at 4°C, the wells were washed 3 times with a solution of PBS/0.05%tween 20, then saturated with a solution of 0.1M Tris 20%, sucrose pH 7.8. The mouse AN-p19C3 hybridoma culture supernatant was added in triplicates. After a 6h incubation, the wells were washed three times with a 5 solution of PBS 0.05%, tween 20 and an anti-mouse antibody coupled with peroxidase (Biosource) diluted to 1/5,000 in PBS, 0.1% BSA, 0.01% Tween 20 was added for an additional 1h30-2h. Binding of the antibody was revealed using ABTS [2,2'-azino-bis(3- ethylbenzthiazoline-6-sulfonic acid)] as a substrate, and the reading was carried out at 405 nm.

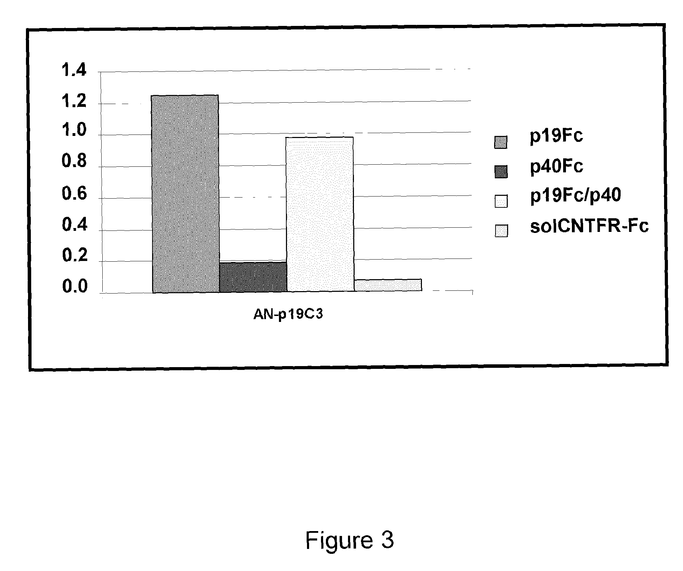

The results are illustrated by Figure 3. They show that AN-p19C3 specifically binds p19 and IL-23, and does not bind p40 or the control protein, soluble CNTFR

### AN-p19C3 vizualisation

Cells of the hybridoma AN-p19C3 producing the antibody, were used for ascites production of the antibody. The mice were treated with an IP. injection of 0.5 ml pristane, 8 days before the injection of hybridoma cells. Two weeks later, the ascites were withdrawn, and the antibody purified by the technique of sequential precipitation with caprylic acid. The caprylic acid precipitates the proteins of a molecular weight lower than 100-120 kDa. The precipitate was spin down, and immunoglobulins present in the supernatant were then precipitated using ammonium sulphate at a 45% final concentration (w/v). These two successive precipitations made it possible to obtain, starting from 2 mice, 30 milligrams of purified antibody.

The secreted AN-p19C3 immunoglobulin is an IgG1 Kappa. The results of analysis by SDS-PAGE show that AN-p19C3 presents the conventional features of IgG (heavy chain 50 kDa, light chain 25 kDa). Data not shown.

Further experiments were performed in order to better characterize the antibody.

### Western blot analysis:

P19 and p40 were tagged with a protein C epitope, and a Flag epitope was linked to the carboxy side of p40 and expressed in mamalian cells (Cos or Heck293 cells ). Samples of p19, p40, IL-12 (p35/p40), IL-23 (p19/p40) (100 ng per leane) were run on SDS-PAGE in reducing conditions, and the blotting was performed onto PVDF membrane for 1h at room temperature. Nonspecific sites were blocked by soaking the membrane in 5% BAS in TBS-Tween 0.1% buffer (2 hr, room temperature). Incubation with 2 µg/ml primary antibody (HPC4 anti-protein C mAb, or anti-Flag antibody for the detection of p40), was performed overnight at +4C. After 3 washes with TBS-Tween, the membrane was incubated with the secondary antibody conjugated with horseradish peroxidase (1:5000) for 2 h at room temperature. The signal was visualized by chemiluminescence using an ECL reagent.

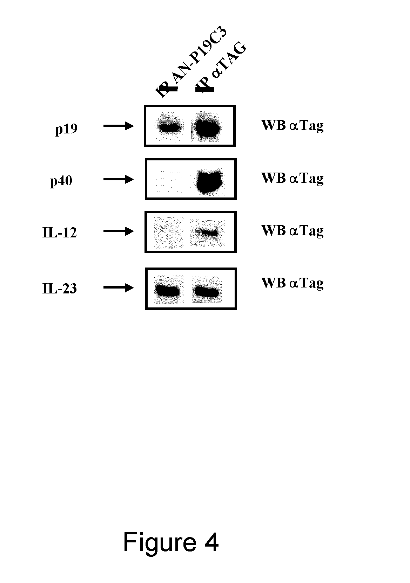

The results are presented in Figure 4. These results show that the AN-p19C3 recognized p19 alone or in the IL-23 context. In contrast, no signal is observed for p40 or IL-12.

### Obtention of the TF23 cell line

The TF1 cell line is an erythroleukemia cell line which proliferation is driven by GM-CSF or eythropietin (*Establishment and characterization of a unique human cell line that proliferates dependently on GM-CSF, IL-3, or erythropoietin.* Kitamura T, et al Cell Physiol. 1989 Aug; 140(2):323-34.).

The inventors detected an expression of IL-12Rβ1 in this cell line, and progressively adapted it, over a period of several months, to grow in the presence of IL-23. This novel and unique cell line able to grow in the presence of IL-23, was termed TF23.

For proliferation assays, TF23 were seeded in 96-well plates at a concentration of 5-10x10³ cells/well in RPMI 1640 medium containing 5% FCS. Serial dilutions of the cytokines tested were performed in triplicate. After a 5-day incubation period, 0,5 µCi of ³HTdr was added to each well for the last 4 hours of the culture and the incorporated radioactivity determined by scintillation counting.

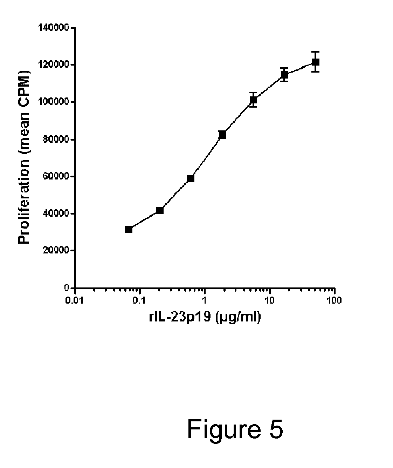

Figure 5 shows a typical curve response of TF23 to IL-23.

### AN-P19C3 mAb inhibits the proliferation response of the TF23 cell line induced by IL-23:

TF23 were seeded in 96-well plates at a concentration of 5-10x10³ cells/well in RPMI 1640 medium containing 5% FCS in the presence of 2 ng/ml IL-23, and serial concentrations of AN-p19 C2 or of AN-p19 C3 antibodies ranging from 0.2 to 50 µg/ml antibody. After a 5-day incubation period, 0,5 Ci of ³HTdr was added to each well for the last 4 hours of the culture and the incorporated radioactivity determined by scintillation counting.

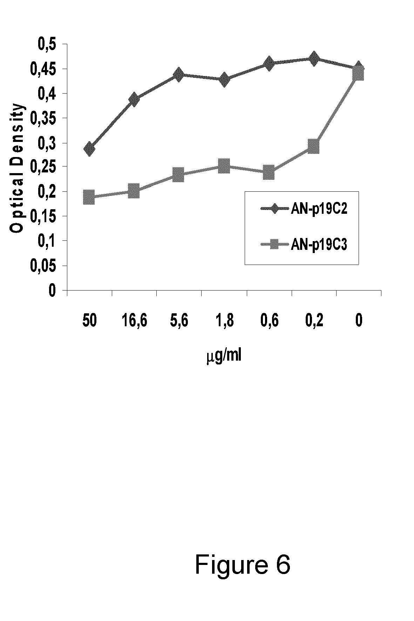

Figure 6 shows a typical neutralization effect of AN-p19C3 antibody towards IL-23. It also shows that another antibody against p19 did not neutralize IL-23 to the same extent.

### Tyrosine phosphorylation analysis:

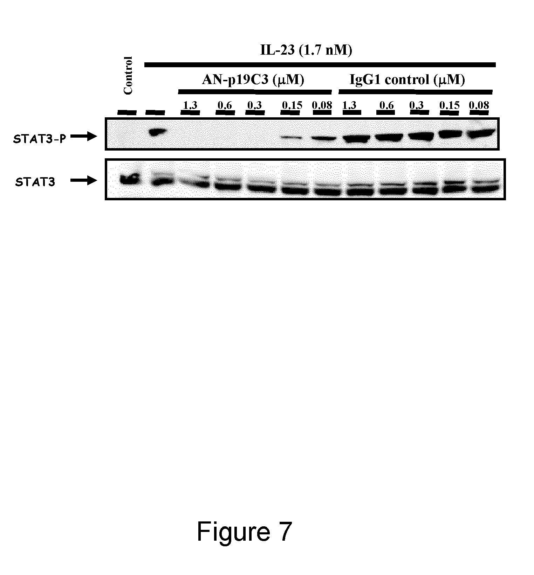

After a 24h serum starvation, TF23 cells were stimulated for 10 min in the presence of IL-23. Cells were next lysed in 10 mM Tris-HCl pH 7.6, 5 mM EDTA, 50 mM NaCl, 30 mM sodium pyrophosphate, 50 mM sodium fluoride, 1 mM sodium orthovanadate, proteinase inhibitors (1µg/ml pepstatin, 2 µg/ml leupeptin, 5 µg/ml aprotinin, 1mM PMSF) and 1% NP40 or Brij 96 depending on the experiments. After pelleting insoluble material and protein standardization, the supernatants were immunoprecipitated overnight. The complexes were then isolated with beads coupled to protein A, submitted to SDS-PAGE and transferred onto an Immobilon membrane (Millipore, Bedford, MA, USA). The membranes were subsequently incubated with a rabbit polyclonal antibody raised against the tyrosine phosphorylated form of STAT-3 (Cell signalling, # 9131 S) at a 1/1,000 dilution, before being incubated with a goat anti rabbit IgG -HRP antibody from Invitrogen (# G21234). The reaction was visualized on an X-ray film using the ECL reagent (Amersham, Les Ullis, France) according to the manufacturer's instructions. The membranes were stripped overnight in 0.1 M glycine-HCl, pH 2.7, and neutralized in 1 M Tris-HCl, pH 7.6, before reblotting with a rabbit polyclonal antibody directed against total STAT-3, and ECL detection, for the loading control of the gel. Figure 7 shows a typical neutralization effect of AN-p19C3 antibody towards STAT-3 phosphorylation in TF23 cells.

### AFFINITY OF THE IL-23 for AN-p19C3 antibody

### Immobilization of the ligands onto the sensor surfaces

To estimate AN-p19C3 affinity constant and the antibody-antigen interactions, surface plasmon resonance (SPR) measurements were performed with a BIAcore 2000 instrument using carboxymethylated dextran CM5 chips (BIAcore, Piscataway, NJ). AN-P19C3 mAb, dilued at 2 µg/ml in 5mM maleate buffer (pH 5.75), was immobilized on the CM5 chips by amine coupling.

### Binding assays and data analysis

IL-23 or IL-12 were diluted in running buffer HBS-EP (O5OIM HEPES pH 7.4 ; 0.15M NaCl, 3mM EDTA and 0.005 % polysorbate 20). Analyte was injected in 3 min, 60 µL/min injections and dissociation was monitored during 10 min. Surface Ab were generated by a 30-s to 1-min injection of glycine-HCL 10mM pH 2. Kinetic rate constants were determined with purified antibodies. Concentrated IL-23 or IL-12 (XXX nM to XXX nM) were injected over the chip surface at a rate of 60 µL/min to collect binding data. Data analyses were carried out with the BIAevaluation 3.0 software.

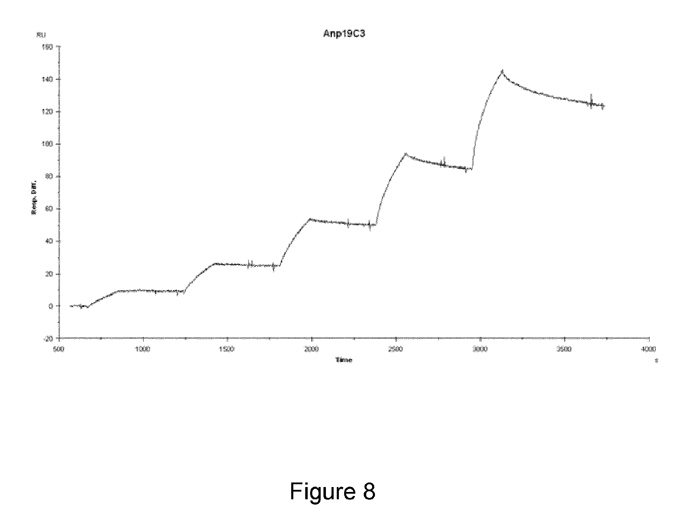

The results are shown on Figure 8. The concentrations of IL-23 and IL-12 are indicated for each curve (Blk=blank). As expected, no binding was observed with the IL-12 molecules whereas a 1.4 nM affinity was measured for IL-23.

### SEQUENCE LISTING

<110> EFFIMUNE INSTITUT NATIONAL DE LA SANTE ET DE LA RECHERCHE MEDICALE UNIVERSITE D'ANGERS
<120> Antibodies directed against p19 subunit of human IL-23 - Use for the preparation of therapeutic molecules
<130> B9526-AD/SDU
<140> EPXXXXXXXX.X
   <141> 2011-10-19
<160> 20
<170> PatentIn version 3.5
<210> 1
   <211> 357
   <212> DNA
   <213> Mus musculus AN-P19C3VH
<220>
   <221> CDS
   <222> (1)..(357)
<400> 1
   cag gtc cag ctt cag cag tct ggg gct gaa ctg gca caa cct ggg gcc      48
   Gln Val Gln Leu Gln Gln Ser Gly Ala Glu Leu Ala Gln Pro Gly Ala
   1               5                   10                  15
   tca gtg aag atg tcc tgc aag gct tct ggc tac acc ttt act agg tac      96
   Ser Val Lys Met Ser Cys Lys Ala Ser Gly Tyr Thr Phe Thr Arg Tyr
               20                  25                  30
   tgg att cac tgg gta aaa cag agg cct gga cag ggt ctg gaa tgg att     144
   Trp Ile His Trp Val Lys Gln Arg Pro Gly Gln Gly Leu Glu Trp Ile
           35                  40                  45
   gga tac att act cct agc act gat tat act gag tac aat cag agg ttc     192
   Gly Tyr Ile Thr Pro Ser Thr Asp Tyr Thr Glu Tyr Asn Gln Arg Phe
       50                  55                  60
   aag gac aag gcc aca ttg act gca gac aaa tcc tcc agt acg gcc tac     240
   Lys Asp Lys Ala Thr Leu Thr Ala Asp Lys Ser Ser Ser Thr Ala Tyr
   65                  70                  75                  80
   atg caa ctg acc agc ctg aca tct gag gac tct gca gtc tat ttc tgt     288
   Met Gln Leu Thr Ser Leu Thr Ser Glu Asp Ser Ala Val Tyr Phe Cys
                   85                  90                  95
   gca aga cac cac gat aac agt cct cac ttt gac tcc tgg ggc caa ggc     336
   Ala Arg His His Asp Asn Ser Pro His Phe Asp Ser Trp Gly Gln Gly
               100                 105                 110
   acc act ctc aca gtc tcc tca                                         357
   Thr Thr Leu Thr Val Ser Ser
           115
<210> 2
   <211> 119
   <212> PRT
   <213> Mus musculus AN-P19C3VH
<400> 2
   Gln Val Gln Leu Gln Gln Ser Gly Ala Glu Leu Ala Gln Pro Gly Ala
   1               5                   10                  15
   Ser Val Lys Met Ser Cys Lys Ala Ser Gly Tyr Thr Phe Thr Arg Tyr
               20                  25                  30
   Trp Ile His Trp Val Lys Gln Arg Pro Gly Gln Gly Leu Glu Trp Ile
           35                  40                  45
   Gly Tyr Ile Thr Pro Ser Thr Asp Tyr Thr Glu Tyr Asn Gln Arg Phe
       50                  55                  60
   Lys Asp Lys Ala Thr Leu Thr Ala Asp Lys Ser Ser Ser Thr Ala Tyr
   65                  70                  75                  80
   Met Gln Leu Thr Ser Leu Thr Ser Glu Asp Ser Ala Val Tyr Phe Cys
                   85                  90                  95
   Ala Arg His His Asp Asn Ser Pro His Phe Asp Ser Trp Gly Gln Gly
               100                 105                 110
   Thr Thr Leu Thr Val Ser Ser
           115
<210> 3
   <211> 27
   <212> DNA
   <213> Mus musculus AN-P19C3VH-CDR1
<220>
   <221> CDS
   <222> (1)..(27)
<400> 3
   tac acc ttt act agg tac tgg att cac                                   27
   Tyr Thr Phe Thr Arg Tyr Trp Ile His
   1               5
<210> 4
   <211> 9
   <212> PRT
   <213> Mus musculus AN-P19C3VH-CDR1
<400> 4
   Tyr Thr Phe Thr Arg Tyr Trp Ile His
   1               5
<210> 5
   <211> 51
   <212> DNA
   <213> Mus musculus AN-P19C3VH-CDR2
<220>
   <221> CDS
   <222> (1)..(51)
<400> 5
   tac att act cct agc act gat tat act gag tac aat cag agg ttc aag        48
   Tyr Ile Thr Pro Ser Thr Asp Tyr Thr Glu Tyr Asn Gln Arg Phe Lys
   1               5                   10                  15
   gac                                                                    51
   Asp
<210> 6
   <211> 17
   <212> PRT
   <213> Mus musculus AN-P19C3VH-CDR2
<400> 6
   Tyr Ile Thr Pro Ser Thr Asp Tyr Thr Glu Tyr Asn Gln Arg Phe Lys
   1               5                   10                  15
   Asp
<210> 7
   <211> 30
   <212> DNA
   <213> Mus musculus AN-P19C3VH-CDR3
<220>
   <221> CDS
   <222> (1)..(30)
<400> 7
   cac cac gat aac agt cct cac ttt gac tcc                                30
   His His Asp Asn Ser Pro His Phe Asp Ser
   1               5                   10
<210> 8
   <211> 10
   <212> PRT
   <213> Mus musculus AN-P19C3VH-CDR3
<400> 8
   His His Asp Asn Ser Pro His Phe Asp Ser
   1               5                   10
<210> 9
   <211> 336
   <212> DNA
   <213> Mus musculus AN-P19C3VL
<220>
   <221> CDS
   <222> (1)..(336)
<400> 9
   gac att gtg ctg acc caa tct cca gct tct ttg gct gtg tct cta ggg        48
   Asp Ile Val Leu Thr Gln Ser Pro Ala Ser Leu Ala Val Ser Leu Gly
   1               5                   10                  15
   cag agg gcc acc atc tcc tgc aga gcc agc gaa agt gtt gat agt tat        96
   Gln Arg Ala Thr Ile Ser Cys Arg Ala Ser Glu Ser Val Asp Ser Tyr
               20                  25                  30
   ggc att agt ttt atg aac tgg ttc caa cag aaa cca gga cag cca ccc       144
   Gly Ile Ser Phe Met Asn Trp Phe Gln Gln Lys Pro Gly Gln Pro Pro
           35                  40                  45
   aaa ctc ctc atc tat gct gca tcc aac caa gga tcc ggg gtc cct gcc       192
   Lys Leu Leu Ile Tyr Ala Ala Ser Asn Gln Gly Ser Gly Val Pro Ala
       50                  55                  60
   agg ttt agt ggc agt ggg tct ggg aca gat ttc agc ctc aac atc cat       240
   Arg Phe Ser Gly Ser Gly Ser Gly Thr Asp Phe Ser Leu Asn Ile His
   65                  70                  75                  80
   cct atg gag gag gat gat acc gca atg tat ttc tgt cag caa agt aag       288
   Pro Met Glu Glu Asp Asp Thr Ala Met Tyr Phe Cys Gln Gln Ser Lys
                   85                  90                  95
   gag gtt cct ctc acg ttc ggt gct ggg acc aag ctg gag ctg aaa cgg       336
   Glu Val Pro Leu Thr Phe Gly Ala Gly Thr Lys Leu Glu Leu Lys Arg
               100                 105                 110
<210> 10
   <211> 112
   <212> PRT
   <213> Mus musculus AN-P19C3VL
<400> 10
   Asp Ile Val Leu Thr Gln Ser Pro Ala Ser Leu Ala Val Ser Leu Gly
   1               5                   10                  15
   Gln Arg Ala Thr Ile Ser Cys Arg Ala Ser Glu Ser Val Asp Ser Tyr
               20                  25                  30
   Gly Ile Ser Phe Met Asn Trp Phe Gln Gln Lys Pro Gly Gln Pro Pro
           35                  40                  45
   Lys Leu Leu Ile Tyr Ala Ala Ser Asn Gln Gly Ser Gly Val Pro Ala
       50                  55                  60
   Arg Phe Ser Gly Ser Gly Ser Gly Thr Asp Phe Ser Leu Asn Ile His
   65                  70                  75                  80
   Pro Met Glu Glu Asp Asp Thr Ala Met Tyr Phe Cys Gln Gln Ser Lys
                   85                  90                  95
   Glu Val Pro Leu Thr Phe Gly Ala Gly Thr Lys Leu Glu Leu Lys Arg
               100                 105                 110
<210> 11
   <211> 45
   <212> DNA
   <213> Mus musculus AN-P19C3VL-CDR1
<220>
   <221> CDS
   <222> (1)..(45)
<400> 11
   aga gcc agc gaa agt gtt gat agt tat ggc att agt ttt atg aac 45
   Arg Ala Ser Glu Ser Val Asp Ser Tyr Gly Ile Ser Phe Met Asn
   1               5                   10                  15
<210> 12
   <211> 15
   <212> PRT
   <213> Mus musculus AN-P19C3VL-CDR1
<400> 12
   Arg Ala Ser Glu Ser Val Asp Ser Tyr Gly Ile Ser Phe Met Asn
   1               5                   10                  15
<210> 13
   <211> 21
   <212> DNA
   <213> Mus musculus AN-P19C3VL-CDR2
<220>
   <221> CDS
   <222> (1)..(21)
<400> 13
   gct gca tcc aac caa gga tcc                                21
   Ala Ala Ser Asn Gln Gly Ser
   1               5
<210> 14
   <211> 7
   <212> PRT
   <213> Mus musculus AN-P19C3VL-CDR2
<400> 14
   Ala Ala Ser Asn Gln Gly Ser
   1               5
<210> 15
   <211> 27
   <212> DNA
   <213> Mus musculus AN-P19C3VL-CDR3
<220>
   <221> CDS
   <222> (1)..(27)
<400> 15
   cag caa agt aag gag gtt cct ctc acg                        27
   Gln Gln Ser Lys Glu Val Pro Leu Thr
   1               5
<210> 16
   <211> 9
   <212> PRT
   <213> Mus musculus AN-P19C3VL-CDR3
<400> 16
   Gln Gln Ser Lys Glu Val Pro Leu Thr
   1               5
<210> 17
   <211> 23
   <212> DNA
   <213> IgG1-REV-VH primer
<400> 17
   ggaaggtgtg cacactgctg gac 23
<210> 18
   <211> 31
   <212> DNA
   <213> MsVHE-FOR-VH primer
<400> 18
   gggaattcga ggtgcagctg caggagtctg g 31
<210> 19
   <211> 21
   <212> DNA
   <213> kappa REV-VL primer
<400> 19
   ggatacagtt ggtgcagcat c 21
<210> 20
   <211> 24
   <212> DNA
   <213> kappa-FOR-VL primer
<400> 20
   gayattgtgm tsacmcarwc tmca 24

## Claims

1. A method for the preparation of hybridoma expressing antibodies which bind the p19 subunit of the native human IL-23 protein and have neutralizing capacity for said native human IL-23 protein, which comprises the steps of immunizing a non-human animal, especially a non-human mammalian, with human IL-23 and, where necessary, boosting said animal with the same immunogen, recovering B cells, especially splenocytes, from the animal responding to immunization and fusing said cells with myeloma cells in conditions enabling the recovery of hybridoma producing the antibodies having the desired binding affinity, **characterized in that** the human IL-23 is in its native form.

2. A method according to claim 1, wherein the myeloma cells are SP2/O mouse cells or rat IR983F cells.

3. A method according to any of claims 1 or 2 , wherein the antibody producing cells and myeloma cells are from same origin, in particular mammalian cells of the same animal.

4. A method for the preparation of antibodies, comprising the preparation of hybridoma according to any of claims 1 to 3 and further comprising recovering from the supernatant of the hybridoma the expressed monoclonal antibodies binding the p19 subunit in native human IL-23, and optionally purifying the same.

5. A method for the expression of antibodies or antigen-binding fragments thereof comprising preparing a cDNA library from the RNA expressed in the hybridoma prepared according to any of claim 1 to 3, selecting the appropriate cDNA from said library and expressing this cDNA in a host cell.

6. A method according to claim 5, wherein the host cell is a eukaryotic cell suitable for a modified glycosylation

7. A method for the preparation of antigen-binding fragments of antibodies consisting in obtaining antigen-binding fragments by enzymatic digestion starting from an antibody obtained by the method of any of claims 4 to 6.

8. A method to obtain antigen-binding fragments of antibodies, wherein the antibodies are obtained by the method of any of claims 4 to 6, and the fragments are expressed in host cells recombined with the nucleic acid sequences encoding the amino acid sequence of said fragments, and optionally wherein said fragments were defined by comparison with sequences of antibodies in accordance with the available data.

9. A method according to any of claims 5 to 8, wherein the antibodies are obtained from hybridoma AN-P19C3 deposited at the CNCM under No I-4539.

10. A method to obtain multivalent antigen-binding fragments consisting in combining antigen-binding fragments obtained by any of claims 5 to 9.

11. A method to obtain antibodies or antigen-binding fragments thereof according to any of claims 4 to 10, further comprising assaying the binding capacity to native human IL-23 or p19 and / or neutralizing capacity of said antibody or fragment.

12. A method to obtain modified antibodies or antigen-binding fragments thereof consisting in modifying the antibody or antigen-binding fragment thereof obtained by any of claims 4 to 11 by humanization, de-immunization and / or rendering it chimeric, to the extent that the resulting fragments keep sufficient antigen-binding capacity and neutralizing capacity for the p19 subunit in native human IL-23.

13. A method according to claim 12, comprising modifying the CDR regions, especially the CDR-3 region, to the extent necessary to de-immunize the antibody.

14. A method according to any of claims 12 or 13, comprising humanizing the antibody or antigen-binding fragment thereof.

15. A method according to claim 14, wherein the antibody or antigen-binding fragment is humanized by substitution of amino acid residue(s) present in its constant region(s) for human amino acid residue(s) having corresponding location in human antibodies according to standard definition and numbering

16. A method according to claim 14 wherein the antibody or antigen-binding fragment is humanized by CDR grafting, and optionally additionally comprising modifying the antibody by framework optimization.

17. A method according to any of claims 12 to 16, comprising rendering the antibody or antigen-binding fragment thereof chimeric by fusion with a protein or conjugation resulting from any suitable form of attachment with a chemical or biological group or a molecule.

18. A method according to claim 17, wherein the antibody or antigen-binding fragment thereof is PEGylated.

## Patentansprüche

1. Verfahren zur Gewinnung von Antikörper exprimierendem Hybridom, welche Antikörper die p19-Untereinheit des nativen, humanen IL-23-Proteins binden und eine neutralisierende Kapazität für dieses native, humane IL-23-Protein besitzen, welches die Schritte der Immunisierung eines nicht-humanen Lebewesens, insbesondere eines nicht-humanen Säugers, mit humanem IL-23 und, wenn erforderlich, Boosten des Lebewesens mit demselben Immunogen, Gewinnen von B-Zellen, insbesondere Splenozyten, von dem auf die Immunisierung antwortenden Lebewesen und Fusionieren der Zellen mit Myeloma-Zellen unter Bedingungen umfasst, welche die Gewinnung von die Antikörper produzierendem Hybridom mit der gewünschten Bindungsaffinität ermöglicht, **dadurch gekennzeichnet, dass** das humane IL-23 in seiner nativen Form vorliegt.

2. Verfahren gemäß Anspruch 1, worin die Myeloma-Zellen SP2/0-Mauszellen oder IR983F-Rattenzellen sind.

3. Verfahren gemäß einem jeden der Ansprüche 1 oder 2, worin die Antikörper produzierenden Zellen und Myeloma-Zellen gleichen Ursprung besitzen, insbesondere Säugerzellen desselben Lebewesens.

4. Verfahren zur Gewinnung von Antikörpern, umfassend die Gewinnung von Hybridom gemäß einem jeden der Ansprüche 1 bis 3 und des Weiteren umfassend das Gewinnen der exprimierten monoklonalen Antikörper, welche die p19-Untereinheit in nativem, humanem IL-23 binden, aus dem Hybridom-Überstand, und gegebenenfalls Reinigen derselben.

5. Verfahren zum Exprimieren von Antikörpern oder Antigen bindenden Fragmenten davon, umfassend die Bereitstellung einer cDNA-Bibliothek aus der RNA, welche in dem Hybridom exprimiert wird, das nach einem jeden der Ansprüche 1 bis 3 gewonnen wird, Auswählen der geeigneten cDNA aus dieser Bibliothek und Exprimieren dieser cDNA in einer Wirtszelle.

6. Verfahren gemäß Anspruch 5, worin die Wirtszelle eine eukariotische Zelle ist, die zu einer modifizierten Glykosilierung geeignet ist.

7. Verfahren zur Gewinnung von Antigen bindenden Fragmenten von Antikörpern, bestehend im Erhalten von Antigen bindenden Fragmenten durch enzymatische Verdauung, ausgehend von einem Antikörper, erhalten durch das Verfahren gemäß einem jeden der Ansprüche 4 bis 6.

8. Verfahren zum Erhalten von Antigen bindenden Fragmenten von Antikörpern, worin die Antikörper durch das Verfahren nach einem jeden der Ansprüche 4 bis 6 erhalten werden und die Fragmente in Wirtszellen exprimiert werden, die mit Nukleinsäuresequenzen rekombiniert sind, welche die Aminosäuresequenz dieser Fragmente kodieren und gegebenenfalls worin diese Fragmente durch Vergleich mit Sequenzen von Antikörpern in Übereinstimmung mit den verfügbaren Daten definiert werden.

9. Verfahren gemäß einem jeden der Ansprüche 5 bis 8, worin die Antikörper aus dem Hybridom AN-P19C3 erhalten werden, welches bei der CNCM unter der Nummer I-4539 hinterlegt ist.

10. Verfahren zum Erhalten multivalenter Antigen bindender Fragmente, bestehend im Kombinieren von Antigen bindenden Fragmenten, erhalten nach einem jeden der Ansprüche 5 bis 9.

11. Verfahren zum Erhalten von Antikörpern oder Antigen bindenden Fragmenten davon gemäß einem jeden der Ansprüche 4 bis 10, des Weiteren umfassend das Untersuchen der Bindungskapazität an natives, humanes IL-23 oder p19 und/oder der Neutralisierungskapazität des Antikörpers oder Fragments.

12. Verfahren zum Erhalten modifizierter Antikörper oder Antigen bindender Fragmente davon, bestehend in der Modifizierung des Antikörpers oder Antigen bindenden Fragments davon, erhalten nach einem jeden der Ansprüche 4 bis 11, durch Humanisieren, Entimmunisieren und/oder Chimärisieren bis zu dem Ausmaß, dass die resultierenden Fragmente eine ausreichende Antigen bindende Kapazität und neutralisierende Kapazität für die p19-Untereinheit in nativem, humanem IL-23 behalten.

13. Verfahren gemäß Anspruch 12, umfassend die Modifizierung der CDR-Bereiche, insbesondere des CDR-3-Bereiches, bis zu dem Ausmaß, das erforderlich ist, um den Antikörper zu entimmunisieren.

14. Verfahren gemäß einem jeden der Ansprüche 12 oder 13, umfassend das Humanisieren des Antikörpers oder Antigen bindenden Fragments davon.

15. Verfahren gemäß Anspruch 14, worin der Antikörper oder das Antigen bindenden Fragment durch Substitution eines Aminosäurerestes/von Aminosäureresten, welche(r) in deren konstantem Bereich/konstanten Bereichen für (einen) humanen Aminosäurerest/Aminosäurereste mit entsprechender Lokalisierung in humanen Antikörpern gemäß Standarddefinition und -nummerierung humanisiert wird.

16. Verfahren gemäß Anspruch 14, worin der Antikörper oder das Antigen bindende Fragment durch CDR-Pfropfung humanisiert wird und optional das Modifizieren des Antikörpers durch Strukturoptimierung umfasst.

17. Verfahren gemäß einem jeden der Ansprüche 12 bis 16, umfassend das Chimärisieren des Antikörpers oder Antigen bindenden Fragmentes davon durch Fusionieren mit einem Protein oder Konjugation, resultierend aus jeder geeigneten Form der Anbindung mit einer chemischen oder biologischen Gruppe oder einem Molekül.

18. Verfahren gemäß Anspruch 17, worin der Antikörper oder das Antigen bindende Fragment davon PEGyliert wird.

## Revendications

1. Procédé pour la préparation d'hybridome exprimant des anticorps qui se lient à la sous-unité p19 de la protéine IL-23 humaine native et qui présentent une capacité de neutralisation de ladite protéine IL-23 humaine native, qui comprend les étapes d'immunisation d'un animal non humain, en particulier d'un mammifère non humain, avec de l'IL-23 humaine et, si nécessaire, de stimulation dudit animal avec le même immunogène, de récupération de cellules B, en particulier de splénocytes, de l'animal répondant à l'immunisation et de fusion desdites cellules avec des cellules de myélome dans des conditions permettant la récupération de l'hybridome produisant les anticorps ayant l'affinité de liaison souhaitée, **caractérisé en ce que** l'IL-23 humaine est sous sa forme native.

2. Procédé selon la revendication 1, dans lequel les cellules de myélome sont des cellules SP2/O de souris ou des cellules IR983F de rat.

3. Procédé selon l'une quelconque des revendications 1 ou 2, dans lequel les cellules productrices d'anticorps et les cellules de myélome sont de la même origine, en particulier des cellules de mammifères du même animal.

4. Procédé pour la préparation d'anticorps, comprenant la préparation de l'hybridome selon l'une quelconque des revendications 1 à 3 et comprenant en outre la récupération à partir du surnageant de l'hybridome des anticorps monoclonaux exprimés se liant à la sous-unité p19 dans l'IL-23 humaine native, et éventuellement leur purification.

5. Procédé pour l'expression d'anticorps ou de fragments de liaison à l'antigène de ceux-ci comprenant la préparation d'une banque d'ADNc à partir de l'ARN exprimé dans l'hybridome préparé selon l'une quelconque des revendications 1 à 3, la sélection de l'ADNc approprié à partir de ladite banque et l'expression de cet ADNc dans une cellule hôte.

6. Procédé selon la revendication 5, dans lequel la cellule hôte est une cellule eucaryote appropriée pour une glycosylation modifiée.

7. Procédé pour la préparation de fragments de liaison à l'antigène des anticorps consistant à obtenir des fragments de liaison à l'antigène par digestion enzymatique à partir d'un anticorps obtenu par le procédé de l'une quelconque des revendications 4 à 6.

8. Procédé pour obtenir des fragments de liaison à l'antigène d'anticorps, dans lequel les anticorps sont obtenus par le procédé de l'une quelconque des revendications 4 à 6, et les fragments sont exprimés dans des cellules hôtes recombinées avec les séquences d'acides nucléiques codant pour la séquence d'acides aminés desdits fragments et éventuellement dans lequel lesdits fragments ont été définis par comparaison avec des séquences d'anticorps selon les données disponibles.

9. Procédé selon l'une quelconque des revendications 5 à 8, dans lequel les anticorps sont obtenus à partir de l'hybridome AN-P19C3 déposé à la CNCM sous le n° I-4539.

10. Procédé pour obtenir des fragments multivalents de liaison à l'antigène consistant à combiner des fragments de liaison à l'antigène obtenus par l'une quelconque des revendications 5 à 9.

11. Procédé pour obtenir des anticorps ou des fragments de liaison à l'antigène de ceux-ci selon l'une quelconque des revendications 4 à 10, comprenant en outre l'évaluation de la capacité de liaison à l'IL-23 ou à la p19 humaine native et/ou de la capacité de neutralisation dudit anticorps ou fragment.

12. Procédé pour obtenir des anticorps ou des fragments de liaison à l'antigène de ceux-ci modifiés consistant à modifier l'anticorps ou le fragment de liaison à l'antigène de celui-ci obtenu par l'une quelconque des revendications 4 à 11 par humanisation, dé-immunisation et/ou en le rendant chimérique, dans la mesure où les fragments résultants conservent une capacité de liaison à l'antigène et une capacité de neutralisation de la sous-unité p19 de IL-23 humaine native suffisantes.

13. Procédé selon la revendication 12, comprenant la modification des régions CDR, en particulier la région CDR-3, dans la mesure nécessaire pour dé-immuniser l'anticorps.

14. Procédé selon l'une quelconque des revendications 12 ou 13, comprenant l'humanisation de l'anticorps ou du fragment de liaison à l'antigène de celui-ci.

15. Procédé selon la revendication 14, dans lequel l'anticorps ou le fragment de liaison à l'antigène est humanisé par substitution de résidu(s) d'acide(s) aminé(s) présent(s) dans sa(ses) région(s) constante(s) par un(des) résidu(s) d'acide(s) aminé(s) humain(s) ayant un emplacement correspondant dans des anticorps humains selon la définition et la numérotation standard.

16. Procédé selon la revendication 14, dans lequel l'anticorps ou le fragment de liaison à l'antigène est humanisé par greffage de CDR, et comprenant éventuellement, en outre, la modification de l'anticorps par l'optimisation de la structure.

17. Procédé selon l'une quelconque des revendications 12 à 16, comprenant de rendre chimérique l'anticorps ou le fragment de liaison à l'antigène de celui-ci par fusion avec une protéine ou conjugaison résultant de toute forme de fixation appropriée avec un groupe chimique ou biologique ou une molécule.

18. Procédé selon la revendication 17, dans lequel l'anticorps ou le fragment de liaison à l'antigène de celui-ci est PEGylé.
